Europäisches Patentamt

European Patent Office

Office européen des brevets

(11) Veröffentlichungsnummer: **0 263 462 B1**

(12) **EUROPÄISCHE PATENTSCHRIFT**

(45) Veröffentlichungstag der Patentschrift: 27.11.91

(21) Anmeldenummer: 87114503.3

(22) Anmeldetag: 05.10.87

(51) Int. Cl.⁵: **C07C 209/60**, C07C 211/07, B01J 29/04

(54) Verfahren zur Herstellung von Aminen.

(30) Priorität: 08.10.86 DE 3634247

(43) Veröffentlichungstag der Anmeldung:
13.04.88 Patentblatt 88/15

(45) Bekanntmachung des Hinweises auf die
Patenterteilung:
27.11.91 Patentblatt 91/48

(84) Benannte Vertragsstaaten:
AT BE DE ES FR GB IT LU NL

(56) Entgegenhaltungen:
EP-A- 0 013 630
EP-A- 0 132 736
EP-A- 0 133 938

(73) Patentinhaber: BASF Aktiengesellschaft
Carl-Bosch-Strasse 38
W-6700 Ludwigshafen(DE)

(72) Erfinder: Hölderich, Wolfgang, Dr.
Mannheimer Strasse 18c
W-6710 Frankenthal(DE)
Erfinder: Taglieber, Volker, Dr.
Franz-Liszt-Strasse 15
W-6904 Eppelheim(DE)
Erfinder: Pohl, Hans Henning, Dr.
Mandelring 221
W-6730 Neustadt(DE)
Erfinder: Kummer, Rudolf, Dr.
Kreuzstrasse 6
W-6710 Frankenthal(DE)
Erfinder: Baur, Karl Gerhard, Dr.
Ottweilerstrasse 22
W-6700 Ludwigshafen(DE)

## Beschreibung

Die Erfindung betrifft ein Verfahren zur Herstellung von Aminen durch Anlagerung von Ammoniak oder analog reagierenden Aminen an Olefine.

Bei der Addition von Ammoniak oder analog reagierenden Aminen an Olefine handelt es sich um eine seit langem bekannte und vielfach beschriebene Reaktion. Eine technische Realisierung ist bislang nicht erfolgt, da nach den bekannten Verfahren nur ungenügende Selektivitäten und/oder Katalysator-Standzeiten erreicht werden, wie beispielsweise nach US-PS 2 623 061, US-PS 2 422 631, US-PS 2 501 556 und US-PS 3 412 158. Auch das in US-PS 4 307 250 beschriebene Verfahren eignet sich nicht für die Durchführung im technischen Maßstab. Die als Katalysator verwendeten Alumino-Zeolithe begünstigten starke Polymerbildung und nachfolgende Verkokung, die den Katalysator schnell desaktiviert.

Aus EP-OS 133 938 und EP-OS 132 736 sind Verfahren bekannt, bei denen Bor-, Alumino- und Eisensilikatzeolithe für die Herstellung von Aminen aus Olefinen verwendet werden und auf die Möglichkeit der Dotierung dieser Zeolithe mit Alkali-, Erdalkali- und Übergangsmetallen hingewiesen wird.

Die Erfindung betrifft eine Weiterentwicklung dieses Verfahrens durch die man bessere Umsätze, höhere Selektivitäten und höhere Standzeiten gegenüber undotierten Katalysatoren erreicht.

Es wurde gefunden, daß man hohe Standzeiten und hohe Selektivitäten der Katalysatoren bei der Herstellung von Aminen aus Olefinen und Ammoniak oder primären oder sekundären Aminen bei Temperaturen zwischen 80°C und 400°C und bei Drücken zwischen 40 und 700 bar in Gegenwart eines zeolithischen Katalysators erzielt, wenn man Olefin und Ammoniak oder primäre oder sekundäre Amine oder Gemische derselben in Gegenwart eines Cr-haltigen Borosilitat- oder Cr-haltigen Eisensilikat-Zeolithen des Pentasil-Typs als Katalysator umsetzt, das erhaltene Amin abtrennt und die nichtumgesetzten Einsatzstoffe zurückführt.

Durch die erfindungsgemäße Verwendung der chromdotierten Katalysatoren werden bereits mit niedrigem Ammoniak- bzw. Amin-Überschuß vergleichsweise hohe Selektivitäten an gewünschtem Reaktionsprodukt gegenüber nicht dotierten und bisher bekannten dotierten Katalysatoren erzielt und die Dimerisierung und/oder Oligomerisierung der eingesetzten Olefine vermieden.

Eine Ausführungsform des Verfahrens besteht darin, daß man Ammoniak und/oder Amine zusammen mit Olefinen im molaren Verhältnis von 1:1 bis 5:1 gemischt einem Festbett- oder Wirbelbettreaktor zuführt und bei einem Druck von 40 bis 700 bar, insbesondere 200 bis 300 bar und einer Temperatur von 80 bis 400°C, insbesondere 250 bis 350°C in der Gasphase oder im überkritischen Zustand umsetzt. Eine andere Ausführungsform besteht darin, daß die Reaktion in Flüssigphase bei einem Druck von 40 bis 80 bar und einer Temperatur von 60°C bis 120°C in einem Rührkessel, einem Fest-/Flüssigkeits-Fließbett oder einem Strömungsrohr durchgeführt wird.

Aus dem Reaktionsaustrag wird das gewünschte Produkt mit Hilfe bekannter Methoden, beispielsweise Destillation oder Extraktion, erhalten und nötigenfalls mittels weiterer Trennoperationen auf die gewünschte Reinheit gebracht. Die nichtumgesetzten Eingangsstoffe werden in den Reaktor zurückgeführt.

Man kann einfach oder mehrfach ungesättigte Olefine mit 2 bis 10 C-Atomen bzw. deren Mischungen als Ausgangsstoffe verwenden. Wegen der geringer ausgeprägten Polymerisationsneigung eignen sich Monoolefine besser als Di- und Polyolefine, doch können auch diese mit Hilfe höherer Ammoniak-bzw. Aminüberschüsse selektiv umgesetzt werden. Die Lage des Gleichgewichts und damit der Umsatz zum gewünschten Amin ist sehr stark vom gewählten Reaktionsdruck abhänigig. Hoher Druck begünstigt das Additionsprodukt, doch stellt im allgemeinen aus technischen Gründen der Druckbereich bis 300 bar das Optimum dar. Die Selektivität der Reaktion wird auch neben dem gewählten Ammoniak-/Amin-Überschuß und dem Katalysator, in hohem Maß durch die Temperatur beeinflußt. Zwar nimmt die Reaktionsgeschwindigkeit der Additionsreaktion mit steigender Temperatur stark zu, doch werden konkurrierende Crack- und Rekombinationsreaktionen des Olefins gleichzeitig gefördert. Die Lage des Temperaturoptimums bezüglich Umsatz und Selektivität ist von der Konstitution des Olefins des eingesetzten Amins und des Katalysators abhängig und liegt meist im Bereich von 250°C bis 350°C. Die Verweilzeit ist abhängig von den Einsatzstoffen und liegt zweckmäßigerweise im Bereich zwischen Bruchteilen einer Sekunde und wenigen Minuten.

Als Katalysatoren für die Aminierung von Olefinen verwendet man Cr-haltige Borosilikat- oder Cr-haltige Eisensilikat-Zeolithe des Pentasiltyps oder deren Gemische.

Der Borosilikatzeolith kann z.B. bei 90 bis 200°C unter autogenem Druck synthetisiert werden, indem man eine Borverbindung z.B. $H_3BO_3$, mit einer Siliciumverbindung, vorzugsweise hochdispersem Silciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Geeignet sind z.B. die isotaktischen Zeolithe nach DE-OS 2 006 471. Diese Borosilikatzeolithe können auch hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Geeignete Eisensilikatzeolithe erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkal- oder Erdalkalizusatz bei 100 bis 220°C unter autogenem Druck.

Die so hergestellten Boro- und/oder Eisensilikatzeolithe oder deren Gemische können nach ihrer Isolierung, Trocknung bei 100 bis 160°C, vorzugsweise 110°C und Calcinierung bei 450 bis 550°C, vorzugsweise 500°C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 95 : 5, bevorzugt 75 : 25, Siliciumdioxid, bevorzugt hochdispersem $Al_2O_3$, $TiO_2$, hochdisperses $TiO_2$, $ZrO_2$ sowie Ton. Nach der Verformung werden die Extrudate oder Preßlinge bei 110°C/16 h getrocknet und bei 500°C/16 h calciniert.

Man erhält auch sehr aktive und selektiv wirkende Katalysatoren, wenn man isolierte Eisen- bzw. Borosilikatzeolithe direkt nach der Trocknung verformt und erst nach der Verformung einer Calcinierung unterwirft. Die hergestellten Eisen- und Borosilikatzeolithe können in reiner Form, ohne Binder, als Stränge oder Tabletten eingesetzt werden, wobei als Verformungs-oder Peptisierungshilfsmittel z.B. Ethylcellulose, Stearinsäure, Kartoffelstärke, Ameisensäure, Oxalsäure, Essigsäure, Salpetersäure, Ammoniak, Amine, Kieselsäure und Graphit oder deren Gemische verwendet werden können.

Liegt der Zeolith aufgrund der Art seiner Herstellung nicht in der katalytisch aktiven, aciden H-Form vor, sondern z.B. in der Na-Form, dann kann man diese durch Ionenaustausch z.B. mit Ammoniumionen und anschließende Calcinierung oder durch Behandlung mit Säuren vollkommen oder partiell in die gewünschte H-Form überführen.

Die so erhaltenen Zeolithe werden mit Cr dotiert. Zweckmäßigerweise führt man die Dotierung so durch, daß man z.B. den verformten Zeolithen in einem Steigrohr vorlegt und bei 20 bis 100°C z.B. eine wäßrige oder alkalische oder aminhaltige Lösung einer Chromverbindung z.B. eines Chromhalogenids oder eines Chromnitrats überleitet. Ein derartiger Ionenaustausch kann z.B. an der Wasserstoff-, Ammonium- und Alkaliform des Zeolithen vorgenommen werden. Eine weitere Möglichkeit der Metalldotierung besteht z.B. darin, daß man das zeolithische Material mit einem Halogenid, einem Nitrat oder einem Oxid des Chroms in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform der Chromdotierung besteht z.B. darin, daß man $Cr(NO_3)_3$ in Wasser löst. Mit dieser Lösung wird der verformte oder unverformte Zeolith eine gewisse Zeit, etwa 30 Minuten, getränkt. Die eventuell überstehende Lösung wird am Rotationsverdampfer von Wasser befreit. Danach wird der getränkte Zeolith bei etwa 150°C getrocknet und bei etwa 550°C calciniert. Dieser Tränkvorgang kann mehrmals hintereinander vorgenommen werden, um den gewünschten Chromgehalt einzustellen.

Auch ist es möglich, z.B. eine wäßrige oder alkoholische oder ammoniakalische $Cr(NO_3)_3$-Lösung herzustellen und darin den reinen pulverförmigen Zeolithen bei 40 bis 100°C unter Rühren ca. 24 h aufzuschlämmen. Nach Abfiltrieren, Trocknen bei etwa 150°C und Calcinierung bei etwa 500°C kann das so gewonnene zeolithische Material mit oder ohne Bindemittel zu Strängen, Pellets oder Wirbelgut weiterverarbeitet werden.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber z.B. eine wäßrige oder ammoniakalische $Cr(NO_3)_3$-Lösung bei leicht erhöhter Temperatur zwischen 30 und 80°C im Kreislauf 15 bis 20 h leitet. Danach wird mit Wasser ausgewaschen, bei etwa 150°C getrocknet und bei etwa 550°C calciniert.

Eine Nachbehandlung der dotierten Zeolithe mit Wasserstoff kann vorteilhaft sein.

Wenn bei der erfindungsgemäßen Verwendung der zeolithischen Katalysatoren eine durch Koksabscheidung bedingte Desaktivierung eintritt, empfiehlt es sich, die Zeolithe durch Abbrennen der Koksablagerung mit Luft oder mit einem Luft-/$N_2$-Gemisch bei 400 bis 550°C, bevorzugt 500°C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die hier beschriebenen Katalysatoren können wahlweise als 2 bis 4 mm-Stränge oder als Tabletten mit 3 bis 5 mm Durchmesser oder als Splitt mit Teilchengrößen von 0,1 bis 0,5 mm oder als Wirbelkontakt eingesetzt werden.

Schwerflüchtige oder feste Edukte werden in gelöster Form z.B. in THF-, , Toluol- oder Petrolether-Lösung zum Einsatz gebracht. Generell ist eine Verdünnung des Eduktes mit derartigen Lösungsmitteln

EP 0 263 462 B1

oder mit Inertgasen wie $N_2$, Ar, He, $H_2O$-Dampf möglich.

Beispiele 1 - 4

Die Reaktionen werden unter isothermen Bedingungen in einem Rohrreaktor (Wendel, 0,6 cm Innendurchmesser, 90 cm Länge) durchgeführt. Die Reaktionsprodukte werden durch übliche Methoden abgetrennt und charakterisiert. Die quantitative Bestimmung der Reaktionsprodukte und der Ausgangsprodukte erfolgt gaschromatographisch.

Katalysator A (erfindungsgemäß)

Ein Borosilikatzeolith des Pentasil-Typs wird in einer hydrothermalen Synthese aus 640 g hochdispersem $SiO_2$, 122 g $H_3BO_3$, 8000 g 1,6-Hexandiamin-Lösung (Mischung 50 : 50 Gew.-%) bei 170° C unter autogenem Druck in einem Rührautoklaven hergestellt. Nach Abfiltrieren und Auswaschen wird das kristalline Reaktionsprodukt bei 100° C/24 h getrocknet und bei 500° C/24 h calciniert. Dieser Borosilikatzeolith setzt sich zusammen aus 94,2 Gew.-% $SiO_2$ und 2,3 Gew.-% $B_2O_3$.
Mit diesem Material werden durch Verformen mit einem Verformungshilfsmittel 2 mm-Stränge hergestellt, die bei 110° C/16 h getrocknet und bei 500° C/24 h calciniert werden.
Die Dotierung dieser Stränge erfolgt durch Imprägnieren mit einer wäßrigen $Cr(NO_3)_3$-Lösung. Nach 30minütiger Behandlung im Kolben am Rotationsverdampfer wird das überstehende Lösungsmittel abgesaugt. Nach Trocknung bei 130° C/2 h und bei 540° C/2 h beträgt der Chromgehalt des Katalysators A 1,9 Gew.-%.

Katalysator B (erfindungsgemäß)

Aus dem wie bei Katalysator A hergestellten Borosilikatzeolithpulver werden durch Verformen mit Boehmit im Gewichtsverhältnis 60 : 40 2 mm-Stränge hergestellt, die bei 110° C/16 h getrocknet und bei 500° C/24 h calciniert werden. Diese Stränge werden, wie bei Katalysator A beschrieben, mit Chrom dotiert. Der Chromgehalt des Katalysators B beträgt 2,1 Gew.-%.

Katalysator C (Vergleichskatalysator)

Katalysator C entspricht Katalysator B jedoch ohne Chrom-Dotierung. Die mit Katalysator A-C erhaltenen Versuchsergebnisse sind in Tabelle 1 zusammengestellt. Die Reaktionsbedingungen sind 300° C, 300 bar, Isobutylen : 1 : 1,3 molar und eine Belastung WHSV = 5 $h^{-1}$.

Tabelle 1

| Beispiel | 1 | 2 | 3 |
|---|---|---|---|
| Katalysator | A | B | C |
| Umsatz von Isobuten | 15,4 % | 14,4 % | 13,1 % |
| Selektivität tert.-Butylamin | > 98 % | > 98 % | 96,9 % |

Beispiel 4

Für eine kontinuierliche Herstellung von tert.-Butylamin wird ein Hochdruck-Reaktor mit 2 m Länge und 24 mm Innendurchmesser eingesetzt, der mittels einer Alublockheizung beheizt und mit dreifacher Innentemperaturmessung sowie mit einer Druckhaltung ausgestattet ist. Direkt an den Reaktor anschließend befinden sich eine Destillationskolonne zum Abtrennen der Wertprodukte und eine Rückführung des nicht umgesetzten Olefins und Ammoniaks. Es werden 900 ml des Katalysators B eingebaut. Der Zulauf des Olefins und $NH_3$ erfolgt von oben. Ein Gemisch aus Isobutylen und Ammoniak (1 : 2 molar) wird bei 300° C, 300 bar und einer Belastung von 5 kg/l Katalysator und Stunde umgesetzt. Katalysator B zeigt nach dreimonatiger Laufzeit unveränderte Aktivität und Selektivität.

**Patentansprüche**

4

**1.** Verfahren zur Herstellung von Aminen aus Olefinen und Ammoniak oder primären oder sekundären Aminen bei Temperaturen zwischen 80°C und 400°C bei Drücken zwischen 40 und 700 bar in Gegenwart eines zeolithischen Katalysators, dadurch gekennzeichnet, daß man Olefin und Ammoniak oder primäre und sekundäre Amine oder Gemische derselben in Gegenwart eines Cr-haltigen Borosilikat- oder Cr-haltigen Eisensilikat-Zeolithen des Pentasil-Typs als Katalysator umsetzt, das erhaltene Amin abtrennt und die nichtumgesetzten Einsatzstoffe zurückführt.

**2.** Verfahren nach Anspruch 1, dadurch gekennzeichnet, daß der Cr-Gehalt zwischen 0.05 Gew.-% und 10 Gew.-% liegt.

## Claims

**1.** A process for the preparation of an amine from an olefin and ammonia or primary or secondary amine at from 80 to 400°C under from 40 to 700 bar in the presence of a zeolite catalyst, wherein the olefin and ammonia or primary or secondary amine, or a mixture of these, is reacted in the presence of a Cr-containing borosilicate or Cr-containing iron silicate zeolite of the pentasil type as a catalyst, the amine obtained is isolated and the unconverted starting materials are recycled.

**2.** A process as claimed in claim 1, wherein the Cr content is from 0.05 to 10% by weight.

## Revendications

**1.** Procédé de préparation d'amines à partir d'oléfines et d'ammoniac au d'amines primaires au secondaires, à des températures comprises entre 80°C et 400°C, sous des pressions comprises entre 40 et 700 bar et en présence d'un catalyseur zéolithique, caractérisé en ce qu'on fait réagir une oléfine et de l'ammoniac, des amines primaires et secondaires au des mélanges de ceux-ci en présence, en tant que catalyseur, d'une zéolithe de borosilicate contenant Cr ou de ferrosilicate contenant Cr du type pentasil, an sépare l'amine obtenue et on recycle les substances de départ n'ayant pas réagi.

**2.** Procédé selon la revendication 1, caractérisé en ce que la teneur en Cr se situe entre 0,05% en poids et 10% en poids.